# EUROPÄISCHE PATENTSCHRIFT

(11) **EP 3 499 219 B1**
(45) Veröffentlichungstag und Bekanntmachung des Hinweises auf die Patenterteilung: **05.08.2020**
(21) Anmeldenummer: 17207496.5
(22) Anmeldetag: 14.12.2017
(51) Int. Cl.: G01N 21/359, G01N 21/31, G01N 33/15

(54) **VERFAHREN UND VORRICHTUNG ZUM BESTIMMEN EINES FERTIGARZNEIMITTELS**
METHOD AND DEVICE FOR OPERATING A READY-MADE MEDICATION
PROCÉDÉ ET DISPOSITIF DE DÉTERMINATION D'UNE SPÉCIALITÉ PHARMACEUTIQUE

(43) Veröffentlichungstag der Anmeldung: 19.06.2019
(73) Patentinhaber: Medios Manufaktur GmbH, 10117 Berlin (DE)
(72) Erfinder: Schwarz, Jörg, 25554 Wilster (DE); Mainka, David, 10115 Berlin (DE)
(74) Vertreter: Kilger, Ute

(56) Entgegenhaltungen:
- DE-A1- 10 326 152
- DE-A1-102010 013 335
- US-A1- 2007 008 523
- US-A1- 2009 262 351
- US-A1- 2011 183 426

## Beschreibung

Die Erfindung betrifft ein Verfahren und eine Vorrichtung zum Bestimmen eines Fertigarzneimittels.

### Hintergrund

Fertigarzneimittel können nach dem Herstellen untersucht werden, um zum Beispiel eine korrekte Zusammensetzung des Arzneimittels nachzuprüfen. Auch kann eine messtechnische Prüfung vorgesehen sein, um auf diese Weise Fälschungen oder unreine Nachahmerprodukte hierdurch zu erkennen. Um eine solche Bestimmung des Fertigarzneimittels durchzuführen, wird dieses in einen Probenbehälter angeordnet, um dann die Bestimmung durchzuführen. Handelt es sich um bereits verpackte Fertigarzneimittel, werden diese zuvor aus der Primärverpackung entnommen, um sie dann in dem Probenbehälter, in dem die Probe vermessen wird, anzuordnen.

DE 10 2010 013 335 A1 offenbart eine Vorrichtung zur Kontrolle des Inhalts von Packungseinheiten mit Arzneimitteln. Eine Sensoreinrichtung ist vorgesehen zum Empfang und zur Verarbeitung von durch eine transparente Wand der Packungseinheit tretenden Lichts, z.B. NIR-Strahlung. Die Sensoreinrichtung ermittelt ein für den Inhalt der Packungseinheit charakteristisches Reflexions- oder Absorptionsspektrum und mit einem Referenzspektrum verglichen.

DE 103 26 152 A1 offenbart ein Verfahren zum Quantifizieren der Zusammensetzung eines Produkts mit den folgenden Schritten: Bestrahlen des Produkts mit einer Strahlungsquelle im nahen Infrarotbereich; Empfangen von Strahlung, die durch das Produkt reflektiert oder transmittiert wird, und Bereitstellen eines Ausgangssignals entsprechend der Intensität der empfangenen Strahlung bei einer Anzahl von unterschiedlichen Wellenlängen; Bestimmen auf der Basis des Ausgangssignals mit einem mathematischen Verfahren, ob sich das Produkt innerhalb vorbestimmter Integritätskriterien befindet oder nicht, wobei D das sich bewegende Produkt eine Lösung oder homogene Dispersion enthält und auf Basis des Ausgangssignals der Gehalt mindestens einer in der Dispersion oder Lösung enthaltenen Substanz quantitativ bestimmt wird.

US 2007/008523 A1 offenbart ein Verifizierungssystem für verschreibungspflichtige Medikamente mit einer Datenbank, die eine Vielzahl von Spektralsignaturen enthält, die den identifizierten Arzneimitteln entsprechen. Ein multimodales Multiplex-Sampling (MMS)-Spektrometer erhält ein Spektrum eines zu identifizierenden und zu verifizierenden Arzneimittels. Das Medikament kann sich innerhalb oder außerhalb eines Fläschchens befinden.

US 2009/262351 A1 offenbart einen optischen Sensor, der das Vorhandensein oder Fehlen eines Produkts in einem flüssigen Fördermedium erkennt. Ein Emitter leitet Strahlung in das flüssige Abgabemedium und Detektoren erfassen transmittiertes Licht bei einer Vielzahl von Wellenlängen. Der Ausgang jedes Detektors und Kombinationen von Ausgängen mehrerer Detektoren sind mindestens einem Schwellenwert außerhalb des Produkts zugeordnet.

### Zusammenfassung

Aufgabe der Erfindung ist es, ein Verfahren und eine Vorrichtung zum Bestimmen eines Fertigarzneimittels anzugeben, mit denen das Fertigarzneimittel effizient und materialschonend bestimmt werden kann.

Zur Lösung sind ein Verfahren sowie eine Vorrichtung zum Bestimmen eines Fertigarzneimittels nach den unabhängigen Ansprüchen 1, 12 geschaffen. Ausgestaltungen sind Gegenstand von abhängigen Unteransprüchen.

Nach einem Aspekt ist ein Verfahren zum Bestimmen eines Fertigarzneimittels gemäss Anspruch 1 geschaffen, das Verfahren weist unter anderem Folgendes auf: Bereitstellen eines zu bestimmenden Fertigarzneimittels in einer geschlossenen Primärverpackung, Messen eines NIR-Messspektrums für das zu bestimmende Fertigarzneimittel durch die geschlossenen Primärverpackung hindurch, Vergleichen des NIR-Messspektrums mit einem NIR-Referenzspektrum, welches einem Arzneimittel oder einem Arzneimittel-Wirkstoff zugeordnet ist und Bestimmen, dass das zu bestimmende Fertigarzneimittel das Arzneimittel oder den Arzneimittel-Wirkstoff enthält, wenn das NIR-Messspektrum und das NIR-Referenzspektrum innerhalb einer Fehlergrenze übereinstimmen.

Nach einem weiteren Aspekt ist eine Vorrichtung zum Bestimmen eines Fertigarzneimittels gemäss Anspruch 12 geschaffen. Die Vorrichtung weist eine Messprobenaufnahme auf, die eingerichtet ist, als Messprobe ein zu bestimmendes Fertigarzneimittel in einer geschlossen Primärverpackung aufzunehmen. Es ist eine Messeinrichtung vorgesehen, die eingerichtet ist, ein NIR-Messspektrum für das zu bestimmende Fertigarzneimittel durch die geschlossene Primärverpackung hindurch aufzunehmen. Die Vorrichtung weist weiterhin eine Auswerteeinrichtung auf, die eingerichtet ist, das NIR-Messspektrum mit einem NIR-Referenzspektrum zu vergleichen, welches einem Arzneimittel oder einem Arzneimittel-Wirkstoff zugeordnet ist, und zu bestimmen, dass das zu bestimmende Fertigarzneimittel das Arzneimittel oder den Arzneimittel-Wirkstoff enthält, wenn das NIR-Messspektrum und das NIR-Referenzspektrum innerhalb einer Fehlergrenze übereinstimmen.

Mithilfe der vorgeschlagenen Technologie ist es ermöglicht, das Fertigarzneimittel optisch zu bestimmen oder messtechnisch zu untersuchen, ohne dass dieses aus der Primärverpackung entnommen werden muss. Die geschlossene Primärverpackung bleibt so unbeschädigt. Der Messvorgang wird wegen des Messens durch die geschlossene Primärverpackung hindurch vereinfacht, da zum Beispiel auch ein zufälliger Kontakt mit dem Fertigarzneimittel vermieden ist. Darüber hinaus erfolgt keine mikrobiologische Kontamination.

Es kann vorgesehen sein, für das Fertigarzneimittel mehrere Messungen durchzuführen und die hierbei jeweils gemessenen NIR-Spektren zu mitteln, bevor dann der Vergleich mit dem oder den NIR-Referenzspektren ausgeführt wird. Auch das NIR-Referenzspektrum kann das Ergebnis mehrerer Messungen sein, deren Ergebnis gemittelt wurde.

Wird keine Übereinstimmung der verglichenen NIR-Spektren bestimmt, bedeutet dies, dass die Spektren innerhalb der Fehlergrenze nicht übereinstimmen und sich insoweit im Lichte der Fehlergrenze unterscheiden.

Zur Vorbereitung des Vergleichs des NIR-Messspektrums und des NIR-Referenzspektrums kann vorgesehen sein, die Spektren zu bearbeiten oder aufzubereiten. Zu den Methoden der Spektrenaufbereitung gehört beispielsweise die Vektornormierung. Hierbei werden zum Beispiel zunächst Mittelwerte für die gemessene optische Größe (Absorption, Transmission, Reflexion) bestimmt, um die Mittelwerte dann vom Messspektrum abzuziehen, wobei diese Datenaufbereitung für alle oder einen Teil der spektralen Werte (Wellenlänge, Wellenzahl) durchgeführt werden kann. Alternativ oder ergänzend kann bei der Bearbeitung der Messspektren vorgesehen sein, eine erste, eine zweite und / oder eine dritte Ableitung für die Messwerte zu bestimmen. Auch können andere Normierungsverfahren alternativ oder ergänzend angewendet werden.

Nach einer solchen Datenvorbehandlung, die einen oder mehrere der vorgenannten Schritte umfassen kann, kann eine Auswertung mittels chemometrischer Methoden vorgesehen sein. Es werden das NIR-Messspektrum und das NIR-Referenzspektrum, welche beide einer jeweils gewählten Datenvorbehandlung entsprechend bearbeitet sein können, miteinander verglichen. Für den Spektrenvergleich kann beispielsweise der spektrale Abstand bestimmt werden. Ergibt sich ein spektraler Abstand, welcher einen Grenzwert nicht überschreitet, werden die Spektren als gleich oder ähnlich bestimmt. Für das Bestimmen des spektralen Abstands (spektrale Distanz) sind als solche verschiedene Methoden bekannt. Hierzu gehören zum Beispiel der euklidische Abstand und der Mahalanobis-Abstand.

Das Vergleichen des NIR-Messspektrums mit dem NIR-Referenzspektrum kann weiterhin Folgendes aufweisen oder umfassen: Bestimmen eines ersten spektralen Teilbereichs aus einem beim Messen des NIR-Messspektrums erfassten spektralen Gesamtbereichs, Vergleichen des NIR-Messspektrums und des NIR-Referenzspektrums für den ersten spektralen Teilbereich und Bestimmen, dass das zu bestimmende Fertigarzneimittel das Arzneimittel oder den Arzneimittel-Wirkstoff enthält, wenn das NIR-Messspektrum und das NIR-Referenzspektrum in dem ersten spektralen Teilbereich innerhalb einer ersten Fehlergrenze übereinstimmen. Bei dieser Ausführungsform wird der Vergleich zwischen NIR-Messspektrum und NIR-Referenzspektrum zumindest für den ersten spektralen Teilbereich durchgeführt, welcher einen Ausschnitt des spektralen Gesamtbereichs darstellt, für den das NIR-Messspektrum aufgenommen wurde. In einer alternativen Ausführungsform kann vorgesehen sein, dass der erste spektrale Teilbereich dem spektralen Gesamtbereich entspricht, wenn zum Beispiel das Messen des NIR-Spektrums von vornherein auf den ersten spektralen Teilbereich begrenzt wird, welcher für die Messung des zu bestimmenden Arzneimittels ausgewählt wurde.

Das Vergleichen des NIR-Messspektrums mit dem NIR-Referenzspektrum kann weiterhin Folgendes aufweisen: Bestimmen eines zweiten spektralen Teilbereichs aus einem beim Messen des NIR-Messspektrums erfassten spektralen Gesamtbereichs, wobei der zweite spektrale Teilbereich innerhalb des spektralen Gesamtbereichs von dem ersten spektralen Bereich getrennt ist, Vergleichen des NIR-Messspektrums und des NIR-Referenzspektrums für den zweiten spektralen Teilbereich und Bestimmen, dass das zu bestimmende Fertigarzneimittel das Arzneimittel oder den Arzneimittel-Wirkstoff enthält, wenn das NIR-Messspektrum und das NIR-Referenzspektrum in dem zweiten spektralen Teilbereich innerhalb einer zweiten Fehlergrenze übereinstimmen. Bei dieser Ausführungsform ist in Ergänzung zu der spektralen Analyse mittels Vergleichs im ersten spektralen Bereich ein Vergleich des NIR-Messspektrums und des NIR-Referenzspektrums in mindestens einem zweiten spektralen Teilbereich vorgesehen. Der zweite spektrale Teilbereich ist von dem ersten Spektralbereich verschieden, kann wahlweise aber abschnittsweise zumindest teilweise mit diesem überlappen. So kann vorgesehen sein, dass der zweite spektrale Teilbereich vollständig mit dem ersten Spektralbereich überlappt, beispielsweise derart, dass der zweite spektrale Teilbereich ein Teilbereich innerhalb des ersten spektralen Teilbereichs erfasst.

Die zweite Fehlergrenze kann von der ersten Fehlergrenze verschieden sein. Alternativ können die beiden Fehlergrenzen gleich sein. Bei dieser oder anderen Ausführungsformen kann zum Beispiel ein Grenzwert für den spektralen Abstand (spektrale Distanz) verwendet werden, bespielweise den euklidischen Abstand oder den Mahalanobis-Abstand. Auch können in den verschiedenen Ausführungsformen mehrere Grenzwerte angewendet werden, so dass bestimmt wird, dass das zu bestimmende Fertigarzneimittel das Arzneimittel oder den Arzneimittel-Wirkstoff enthält, wenn das NIR-Messspektrum und das NIR-Referenzspektrum jeweils innerhalb der mehreren Fehlergrenzen übereinstimmen. Wenn mehrere Fehlergrenzen beim Vergleich herangezogen werden, insbesondere betreffend verschiedene spektrale Vergleichsmethoden, kann festgelegt sein, dass das Erfüllen der Fehlergrenz-Bedingung für nur eine Teilmenge der geprüften Fehlergrenzen ausreicht, um zu bestimmen, dass das zu bestimmende Fertigarzneimittel das Arzneimittel oder den Arzneimittel-Wirkstoff enthält.

Das NIR-Messspektrum kann mit mehreren NIR-Referenzspektren aus einer spektralen Referenzbibliothek verglichen werden. In der spektralen Referenzbibliothek kann eine Vielzahl von NIR-Referenzspektren bereitgestellt sein, insbesondere in einer klassifizierten Anordnung. So kann eine Teilbibliothek eine Klassifizierung verschiedener Arzneimittel-Wirkstoffe betreffen, für die jeweils ein oder mehrere NIR-Referenzspektren hinterlegt sind, die den zugeordneten Arzneimittel-Wirkstoff von anderen Arzneimittel-Wirkstoffen unterscheiden. In ähnlicher Weise kann eine Bibliothek für verschiedene Fertigarzneimittel bereitgestellt sein. Als Klassifizierungskriterien können beispielsweise spektrale Bereiche (Wellenlängenbereiche) definiert sein, die für ein bestimmtes Fertigarzneimittel oder einen bestimmten Arzneimittel-Wirkstoff beim Vergleich von NIR-Messspektrum und NIR-Referenzspektrum analysiert werden, um die Übereinstimmung innerhalb der Fehlergrenze zu prüfen.

Das zu bestimmende Fertigarzneimittel kann in einer geschlossenen Einweg-Primärverpackung. Bei der Einweg- oder bei einer Mehrweg-Primärverpackung kann vorgesehen sein, dass die jeweilige Verpackung mit einem Einwegverschluss verschlossen ist.

Das zu bestimmende Fertigarzneimittel kann in einer Sterilverpackung angeordnet sein.

Das zu bestimmende Fertigarzneimittel kann in einem Behälter der geschlossenen Einweg-Primärverpackung, welcher aus Glas und / oder Kunststoff besteht. Der Behälter für das Fertigarzneimittel kann verschiedene Formen aufweisen, beispielsweise eine Glastonne oder eine Blisterverpackung. Im Fall eines Tonnenbehälters aus Glas oder Kunststoff kann vorgesehen sein, die NIR-Strahlen beim Messen durch den Boden des Behälters hindurch einzustrahlen.

Beim Messen des NIR-Messspektrums kann mindestens ein optisches Messverfahren aus der folgenden Gruppe von optischen Messverfahren verwendet werden: Absorptionsmessung, Reflexionsmessung und Transmissionsmessung. Beim Vergleichen von NIR-Messspektrum und NIR-Referenzspektrum können Spektren für eine oder mehrere der optischen Messmethoden einbezogen werden.

Das NIR-Messspektrum wird mit einem NIR-Referenzspektrum verglichen, welches einem als Biosimilar ausgebildeten Arzneimittel-Wirkstoff zugeordnet ist. Biosimilars sind biotechnologisch hergestellte Arzneimittel. Sie können Folgepräparate von Biopharmazeutika sein. Ihr Wirkstoff ist vergleichbar mit dem Wirkstoff des bereits zugelassenen biotechnologisch hergestellten Arzneimittels ("Referenzprodukt"). Häufig sind Biosimilars große, komplexe Moleküle.

Das zu bestimmende Fertigarzneimittel kann den Arzneimittel-Wirkstoff Trastuzumab-Emtansin enthaltend bestimmt werden, wenn das NIR-Messspektrum und Z rein weiteres NIR-Referenzspektrum in einem ersten spektralen Teilbereich von 3500 cm⁻¹ bis 5500 cm⁻¹, alternativ von 4000 cm⁻¹ bis 5000 cm⁻¹ und weiter alternativ von 4150 cm⁻¹ bis 5150 cm⁻¹ innerhalb der ersten Fehlergrenze übereinstimmen.

Das zu bestimmende Fertigarzneimittel kann den Arzneimittel-Wirkstoff Infliximab (Originator) enthaltend bestimmt werden, wenn das NIR-Messspektrum und das NIR-Referenzspektrum in einem ersten spektralen Teilbereich von 3500 cm⁻¹ bis 5500 cm⁻1, alternativ von 4000 cm⁻¹ bis 5000 cm⁻¹ und weiter alternativ von 4550 cm⁻¹ bis 4750 cm⁻¹ innerhalb der ersten Fehlergrenze übereinstimmen.

Das zu bestimmende Fertigarzneimittel kann den Arzneimittel-Wirkstoff nab-Paclitaxel enthaltend bestimmt werden, wenn das NIR-Messspektrum und ein weiteres NIR-Referenzspektrum in einem ersten spektralen Teilbereich von 5600 cm⁻¹ bis 7000 cm⁻¹ und in einem zweiten spektralen Teilbereich von 4000 cm⁻¹ bis 5000 cm⁻¹ innerhalb der ersten Fehlergrenze übereinstimmen.

Das Bestimmen, ob das zu bestimmende Fertigarzneimittel das Arzneimittel oder den Arzneimittel-Wirkstoff enthält, in der hier verwendeten Bedeutung bezieht sich auf das Identifizieren des Arzneimittels und / oder des Arzneimittel-wirkstoffs und / oder das Identifizieren und das Unterscheiden von Abweichungen zwischen gleichen Arzneimitteln und / oder zwischen gleichen Arzneimittel-Wirkstoffen. Die beschriebene Technologie kann verwendet werden zum Identifizieren des Arzneimittels und / oder des Arzneimittel-Wirkstoffs oder zum Identifizieren und Unterscheiden zwischen gleichen Arzneimitteln und / oder zwischen gleichen Arzneimittel-Wirkstoffen.

In Verbindung mit der Vorrichtung zum Bestimmen eines Fertigarzneimittels können die vorangehend erläuterten Ausgestaltungen entsprechend vorgesehen sein.

### Beschreibung von Ausführungsbeispielen

Im Folgenden werden weitere Ausführungsbeispiele unter Bezugnahme auf Figuren einer Zeichnung näher erläutert. Hierbei zeigen:
- Fig. 1: eine schematische Darstellung einer Vorrichtung zum Bestimmen eines Fertigarzneimittels unter Verwendung der NIR-Spektroskopie;
- Fig. 2: eine schematische Darstellung für ein Verfahren zum Bestimmen eines Fertigarzneimittels;

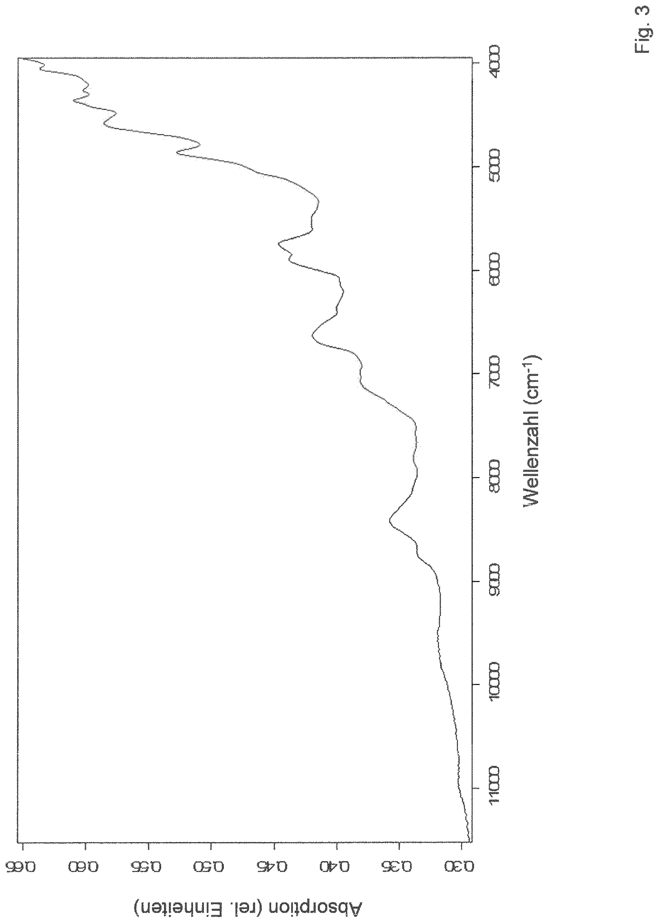

- Fig. 3: eine grafische Darstellung eines NIR-Messspektrums für Abraxane (nab-Paclitaxel), wobei die Absorption und Abhängigkeit von der Wellenzahl dargestellt ist;

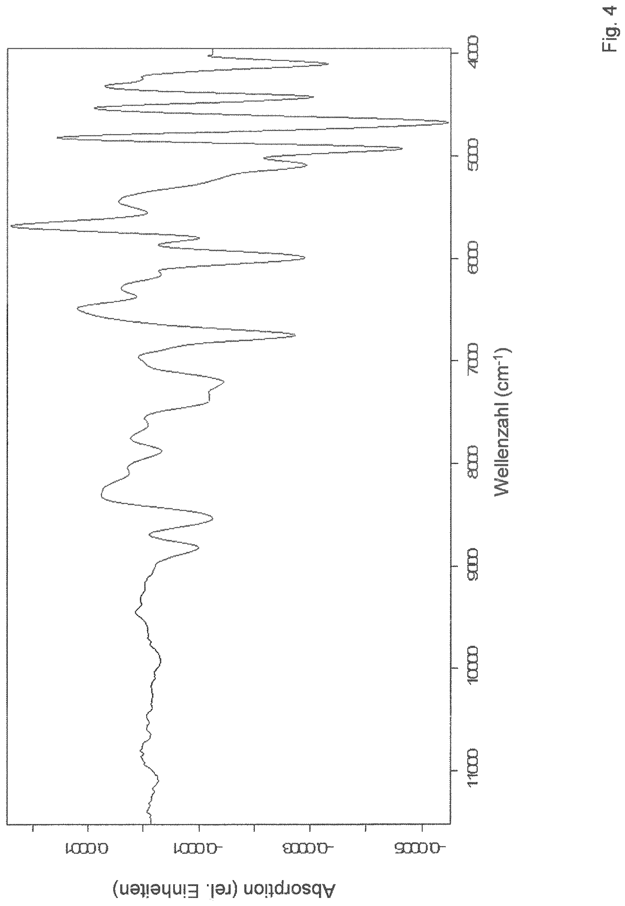

- Fig. 4: eine grafische Darstellung der ersten Ableitung für das NIR-Messspektrum aus Fig. 3;

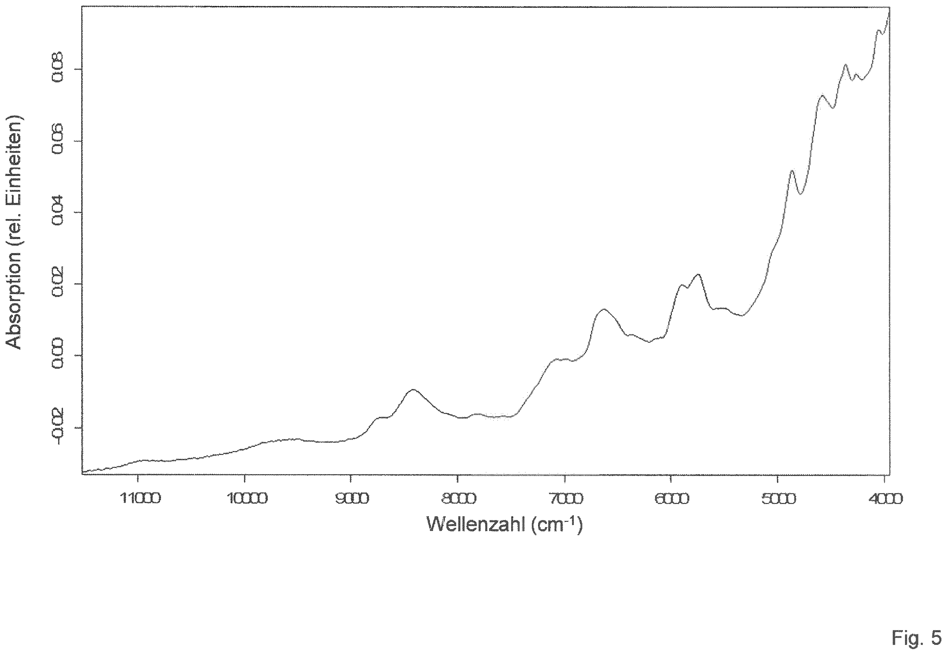

- Fig. 5: eine grafische Darstellung des vektornormierten NIR-Messspektrums aus Fig. 3;
- Fig. 6: eine grafische Darstellung von vorbehandelten NIR-Spektren für vier Fertigarzneimittel und
- Fig. 7: eine grafische Darstellung eines spektralen Ausschnitts für die NIR-Spektren aus Fig. 6.

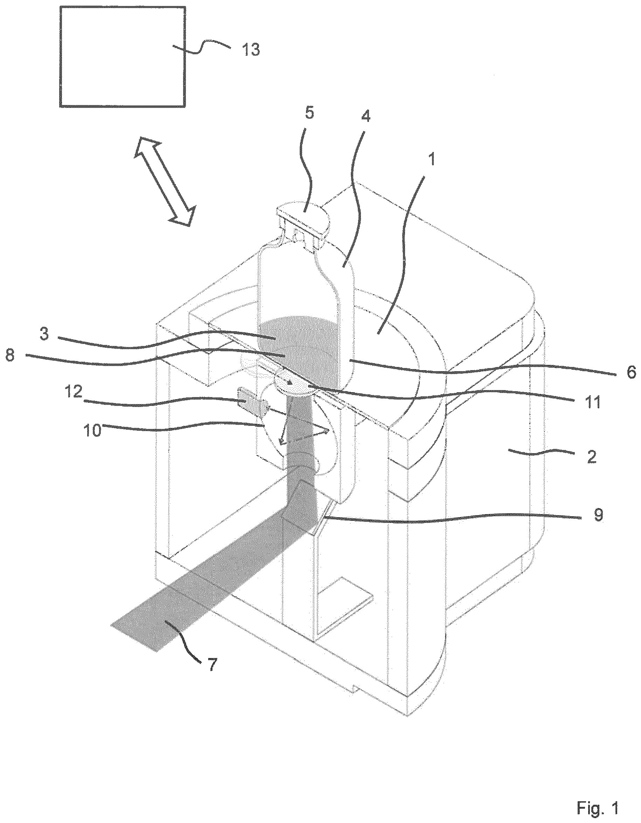

Fig. 1 zeigt eine schematische Darstellung einer Vorrichtung zum Bestimmen eines Fertigarzneimittels mittels NIR-Spektroskopie. Der NIR-Spektralbereich erfasst hierbei Wellenlängen im Bereich von 780 nm bis 2.500 nm (12.800 bis 4.000 cm⁻¹ - Wellenzahl),

Auf einer Probenaufnahme 1 einer Messeinrichtung 2 ist eine Probe 3 eines zu bestimmenden Fertigarzneimittels in einer Primärverpackung 4 angeordnet. Ein Verschluss 5 der Primärverpackung 4 ist ein Einwegverschluss. Die Primärverpackung 4 ist mit einem Behälter 6 gebildet, der zum Beispiel aus Glas oder Kunststoff besteht, beispielweise Braunglas.

Die Probe 3 zu bestimmenden Fertigarzneimittel wird in der geschlossenen Primärverpackung 4 untersucht, indem NIR-Lichtstrahlen 7 über den Bereich des Bodens 8 des Behälters 6 eingestrahlt werden, die die Behälterwandung durchdringen und so zur Probe 3 gelangen. Die NIR-Lichtstrahlen 7 werden hierbei zuvor von einem Spiegel 9 abgelenkt und durch eine Ulbrichtsche Kugel 10 geführt. Für eine Messung wird ein weiterer Spiegel 11 aus der in Fig. 1 gezeigten Stellung heraus verlagert, so dass der Strahlengang auf den Boden 8 des Behälters 6 freigegeben ist.

Infolge des Einstrahlens der NIR-Lichtstrahlen 7 auf die Probe 3 entstehen NIR-Messlichtstrahlen (nicht dargestellt), die aus dem Behälter 6 in die Ulbrichtsche Kugel 10 gelangen und dort zu einem Detektor 12 umgelenkt werden.

Mit dem weiteren Spiegel 11 kann der Strahlengang unterbrochen und geöffnet werden. Die in Fig. 1 gezeigte Unterbrechung kann zum Beispiel für eine Referenzmessung genutzt werden, also eine Messung ohne Probeneinfluss, zum Beispiel zum Verbessern des Signal-Rausch-Verhältnisses. Wenn der weitere Spiegel 11, der beispielsweise als ein Goldspiegel ausgeführt ist, im Strahlengang angeordnet ist, werden die eingestrahlten NIR-Lichtstrahlen 7 gemäß Fig. 1, ohne zur Probe 3 zu gelangen, zurückgeworfen und gelangen zum Detektor 12. Ist der weitere Spiegel 11 seitlich des Strahlengangs angeordnet (nicht dargestellt), kann die Messung an der zu bestimmenden Probe 3 ausgeführt werden.

Es ist weiterhin eine Auswerteeinrichtung 13 vorgesehen, in welcher die mittels des Detektors 12 erfassten NIR-Messspektren verarbeitet und mit NIR-Referenzspektren verglichen werden können. Die Auswerteeinrichtung kann einen oder mehrere Prozessoren und einen Speicher aufweisen, in eine Software-Applikation gespeichert ist, mit das Verarbeiten der gemessenen spektralen Signale ausführbar ist.

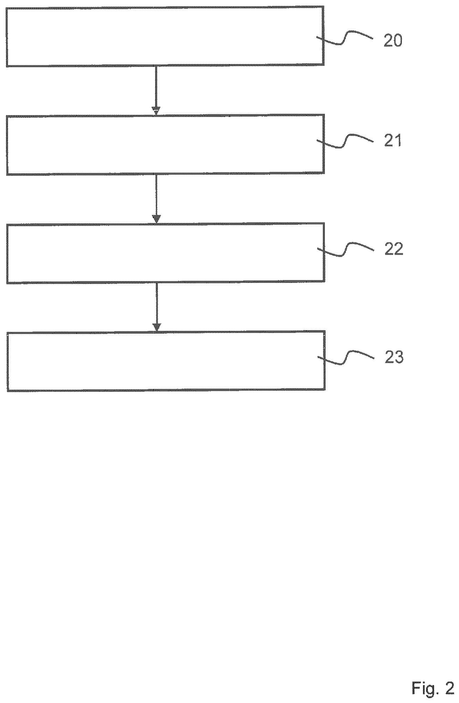

Fig. 2 zeigt eine schematische Darstellung zum Ablauf eines Verfahrens zum Bestimmen eines Fertigarzneimittels. Im Schritt 20 wird das zu bestimmende Fertigarzneimittel in der geschlossenen Primärverpackung 3 bereitgestellt. Im Schritt 21 werden ein oder mehrere NIR-Messspektren gemessen, wobei die NIR-Lichtstrahlen 6 durch die geschlossene Primärverpackung 3 hindurch eingestrahlt werden. Das NIR-Messspektrum wird mit einem NIR-Referenzspektrum verglichen (Schritt 22), welches einem Arzneimittel oder einem Arzneimittel-Wirkstoff zugeordnet ist. Mithilfe der Auswerteeinheit 12 wird bestimmt, ob das zu bestimmende Fertigarzneimittel das Arzneimittel oder den Arzneimittel-Wirkstoff enthält (Schritt 23), wenn das NIR-Messspektrum und das NIR-Referenzspektrum innerhalb einer vorgegebenen Fehlergrenze übereinstimmen.

Beim Auswerten und Vergleichen von NIR-Messspektrum und NIR-Referenzspektrum werden chemometrische Methoden angewendet. Hierzu kann beispielsweise eine Datenvorbehandlung gehören, worauf die chemometrische Auswertung erfolgt. Im Rahmen der Datenvorbehandlung der Spektren kann zum Beispiel eine Vektornormierung ausgeführt werden. Auch können ein oder mehrere Ableitungen für die Spektren berechnet werden. Zur Ableitung der Spektren kann der an sich bekannte Savitzky-Golay-Algorithmus genutzt werden.

Im Rahmen der chemometrischen Auswertung erfolgt dann der Vergleich der (wahlweise vorbehandelten) Spektren. Insbesondere wird ein spektraler Abstand zwischen den zu vergleichenden Spektren bestimmt. Es sind verschiedene Methoden für die Berechnung des spektralen Abstands als solche bekannt. Beispielsweise kann der Euklidische Abstand bestimmt werden. Alternativ oder ergänzend kann vorgesehen sein, den Mahalanobis-Abstand zu bestimmen, um die Ähnlichkeit der zu vergleichenden Spektren innerhalb der jeweils vorgegebenen Fehlergrenze zu ermitteln.

Nachfolgend werden weitere Ausführungsbeispiele erläutert. NIR-Messungen wurden bei Raumtemperatur bestimmt.

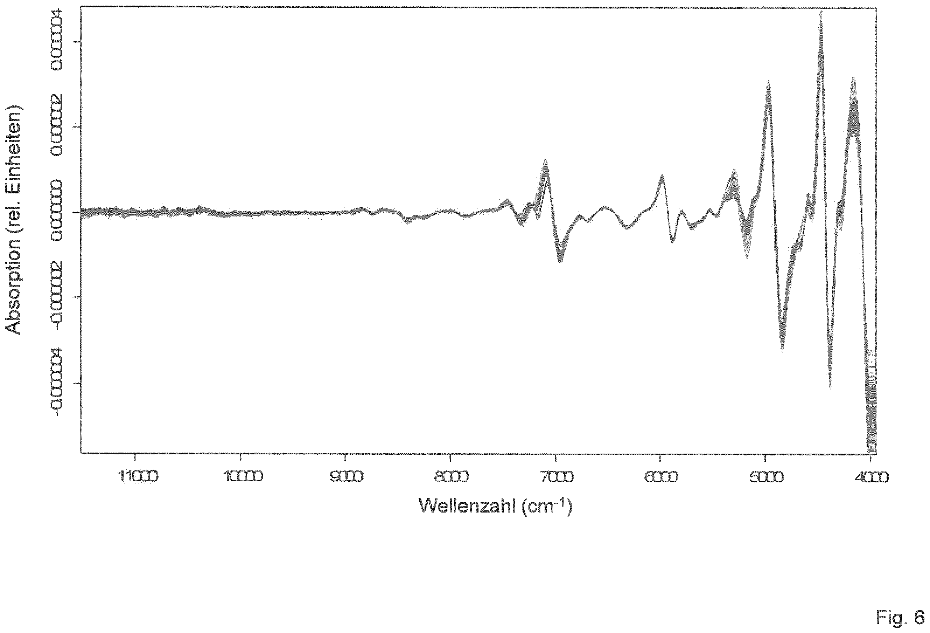

Das Fertigarzneimittel Remicade enthält den Arzneimittel-Wirkstoff Infliximab. Dieses Arzneimittel stellt das Originalprodukt dar. Es ist mindestens ein Biosimilar bekannt, welches von unterschiedlichen Firmen vertrieben wird. Mittels einer Wellenlängenbereichsauswahl von etwa 4592 cm⁻¹ bis etwa 4736 cm⁻¹ kann der Originator vom Biosimilar unterschieden werden (vgl. vorbehandelte, komplette Spektren von vier Fertigarzneimitteln in Fig. 6). Die Ausführungsbeispiele betreffen Remicade und zugehörige Biosimilars: Remsima, Inflectra und Flixabi.

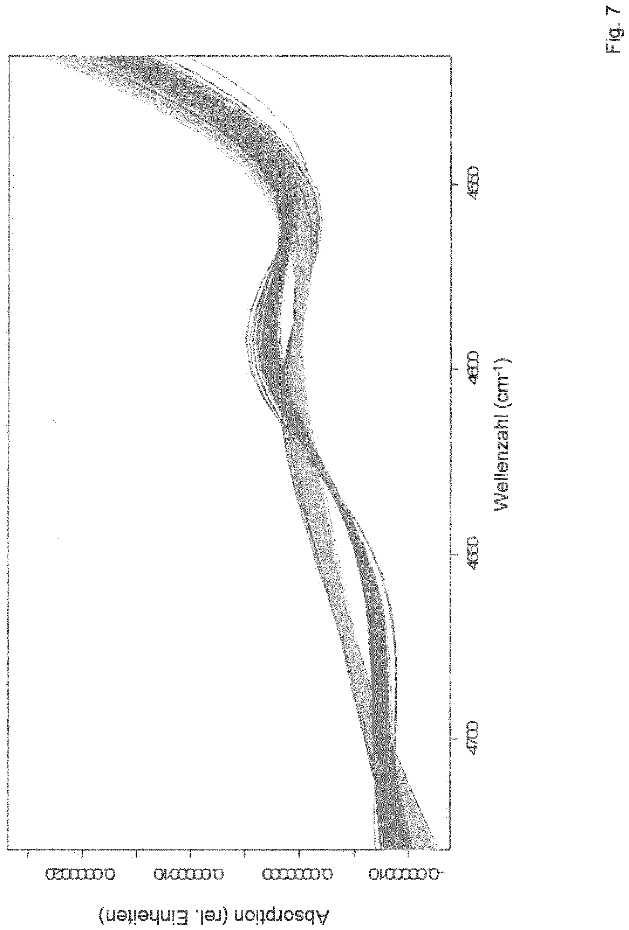

Die in Fig. 7 dargestellten spektralen Unterschiede führen dazu, dass die beiden Wirkstoffe (Originator und Biosimilar), welche sich nach Untersuchungen im Glykosylierungsmuster unterscheiden (vgl. zum Beispiel "arznei-telegramm", 6/15, 46. Jahrgang, 12. Juni 2015), unterschieden werden können. Dies ist üblicherweise nur mit einem aufwendigen LC-MS-Verfahren möglich.

Das Verfahren zum Bestimmen eines Fertigarzneimittels wurde in einem Ausführungsbeispiel zum Bestimmen des Remicades in einem Fertigarzneimittel verwendet. Hierbei wurden für den Vergleich vom NIR-Messspektren und NIR-Referenzspektren aufeinanderfolgend mehrere Vergleichsschritte ausgeführt, in den jeweils ausgewählte spektrale Bereiche verglichen wurden, um die im jeweiligen Schritt vorgesehene Bestimmung oder Identifizierung eines oder mehrerer Stoffe oder den Ausschluss zu erreichen.

Die folgende Tabelle 1 zeigt eine zusammenfassende Übersicht zu den ausgeführten Vergleichsschritten.

**Tabelle 1**

| | | |
|---|---|---|
| Schritte | Beschreibung | Wellenzahlbereich(e) [cm⁻¹] |
| 1 | Auftrennung in Gruppen | 10040-4080 |
| 2 | Bestimmen von Kyprolis und Abtrennen einer weiteren Gruppe chemischdefinierter Arzneimittel-Wirkstoffe | 7760-7200; 6992-5336; 5136-4104 |
| 3 | Bestimmen von Abraxane | 6904-5600; 5000-4000 |
| 4 | Bestimmen von Etopophos | 6968-6584 |
| 5 | Bestimmen von Orencia, Ivemend und Abtrennen von Adcetris und Myocet | 6856-5400; 4992-4776; 4400-4136 |
| 6 | Bestimmen von Entyvio | 6192-6128; 5592-5544; 5096-4952 |
| 7 | Bestimmen von Yondelis, Auftrennung in zwei Gruppen | 7016-5384; 5008-4072 |
| 8 | Auftrennung in zwei Gruppen | 6000-5600; 4904-4144 |
| 9 | Bestimmen von Remicade bzw. Biosimilars | 4736-4592 |

In den Schritten zum spektralen Vergleichen wird stets das NIR-Messspektrum mit einem oder mehreren NIR-Referenzspektren verglichen. Im Schritt 1 erfolgt eine grobe Einteilung in Gruppen A und B, basierend auf dem Wellenzahlbereich von etwa 10.040 cm⁻¹ bis etwa 4.080 cm-¹. Hierbei werden aile NIR-Spektren normiert und einer Datenvorbehandlung unterzogen, um physikalische Einflussfaktoren auf das Spektrum zu minimieren. Zur Vorbereitung des Vergleichs ist hierbei vorgesehen sein, die Spektren aufzubereiten. Zu den Methoden der Spektrenaufbereitung gehört die Vektornormierung. Hierbei werden zunächst Mittelwerte für die gemessene optische Größe bestimmt, um die Mittelwerte dann vom Messspektrum abzuziehen, wobei diese Datenaufbereitung für alle oder einen Teil der spektralen Werte (Wellenlänge, Wellenzahl) durchgeführt werden kann. Alternativ oder ergänzend kann vorgesehen sein, Ableitungen für die Messwerte zu bestimmen. Im Anschluss wird der Spektren-Vergleich ausgeführt. Beispielsweise können hierzu die euklidische Distanz und / oder der Mahalanobis-Abstand verendet werden.

Im nächsten Schritt 2 erfolgt die Abtrennung gegenüber Kyprolis und einer Gruppe von chemisch-definierter Zytostatika. Hierbei werden die störenden Wasserbanden exkludiert, zusätzlich erfolgt eine Vorbehandlung ähnlich der im Schritt 1, mit den individuell optimierten Einstellungen. Die Aufteilung erfolgt mittels des Mahalanobis-Abstands.

In Schritt 3 wird Abraxane identifiziert oder bestimmt, d.h. es wird ausgeschlossen, dass es sich um Abraxane handelt.

Analog werden im Schritt 4 Etopophos, im Schritt 5 Adcetris, Myocet, Ivemend und Orencia und im Schritt 6 Entyvio identifiziert.

In Schritt 7 Yondelis identifiziert oder bestimmt, und es erfolgt eine weitere Auftrennung in zwei Gruppen. In Schritt 8 wird erneut in zwei Gruppen von monoklonalen Antikörpern unterschie-den. In der einen Gruppe befinden sich nur noch die verschiedenen Infliximab-FAM. Diese werden in Schritt 9 mit dem spezifischen Wellenzahlbereich unterschieden.

Die folgende Tabelle 2 zeigt weitere Ausführungsbeispiele für die Bestimmung von Wirkstoffen in Fertigarzneimitteln mittels der beschriebenen Technologie.

**Tabelle 2**

| Lfd. Nr. | Wirkstoff im Arzneimittel | Wellenzahlbereich(e) [cm⁻¹] | Produktbeispiel |
|---|---|---|---|
| 1. | Nab-Paclitaxel | 7100-5500, 5000-4000 | Abraxane 100 mg Celgene |
| 2. | Brentuximab Vedotin | 5000-4000 | Adcetris 50 mg Takeda |
| 3. | Pemetrexed | 6300-5900, 5200-4900 | Alimta 500 mg Lilly |
| 4. | Belimumab | 5900-5300 | Benlysta 120 mg gsk |
| 5. | Belimumab | 5900-5300 | Benlysta 400 mg gsk |
| 6. | Vedolizumab | 6300-6000, 5600-4800 | Entyvio 300 mg Takeda |
| 7. | Infliximab (Biosimilar) | 4800-4300 | Flixabi 100 mg SamsungBioepis |
| 8. | Trastuzumab | 5900-5300 | Herceptin 150 mg Roche |
| 9. | Infliximab (Biosimilar) | 4800-4300 | Inflectra 100 mg Hospira |
| 10. | Trastuzumab Emtansin | 4900-4400 | Kadcyla 100 mg Roche |
| 11. | Trastuzumab Emtansin | 4900-4400 | Kadcyla 160 mg Roche |
| 12. | Pembrolizumab | 4900-4400 | Keytruda 50 mg MSD |
| 13. | Doxorubicin | 5000-4000 | Myocet 50 mg TEVA |
| 14. | Abatacept | 7000-5300, 5100-4000 | Orencia 250 mg b-ms |
| 15. | Infliximab (Original) | 4800-4300 | Remicade 100 mg MSD |
| 16. | Infliximab (Biosimilar) | 4800-4300 | Remsima 100 mg Celltrion |
| 17. | Bortezomib | 7100-6400, 6200-5800, 5300-4900 | Velcade 3,5 mg Janssen |
| 18. | Azacitidin | 6300-5900, 5200-4900 | Vidaza 100 mg Celgene |

Die in der vorstehenden Beschreibung, den Ansprüchen sowie der Zeichnung offenbarten Merkmale können sowohl einzeln als auch in beliebiger Kombination für die Verwirklichung der verschiedenen Ausführungen von Bedeutung sein.

## Patentansprüche

1. Verfahren zum Bestimmen eines Fertigarzneimittels, aufweisend:
- Bereitstellen eines zu bestimmenden Fertigarzneimittels in einer geschlossenen Primärverpackung;
- Messen eines NIR-Messspektrums für das zu bestimmende Fertigarzneimittel durch die geschlossenen Primärverpackung hindurch;
- Vergleichen des NIR-Messspektrums mit einem NIR-Referenzspektrum, welches einem Arzneimittel oder einem Arzneimittel-Wirkstoff zugeordnet ist; und
- Bestimmen, dass das zu bestimmende Fertigarzneimittel das Arzneimittel oder den Arzneimittel-Wirkstoff enthält, wenn das NIR-Messspektrum und das NIR-Referenzspektrum innerhalb einer Fehlergrenze übereinstimmen,
**dadurch gekennzeichnet, dass** beim Vergleichen des NIR-Messspektrums mit dem NIR-Referenzspektrum ein NIR-Referenzspektrum verwendet wird, welches einem als Biosimilar ausgebildeten Arzneimittel-Wirkstoff zugeordnet ist.

2. Verfahren nach Anspruch 1, **dadurch gekennzeichnet, dass** das Vergleichen des NIR-Messspektrums mit dem NIR-Referenzspektrum weiterhin Folgendes aufweist:
- Bestimmen eines ersten spektralen Teilbereichs aus einem beim Messen des NIR-Messspektrums erfassten spektralen Gesamtbereichs;
- Vergleichen des NIR-Messspektrums und des NIR-Referenzspektrums für den ersten spektralen Teilbereich und
- Bestimmen, dass das zu bestimmenden Fertigarzneimittel das Arzneimittel oder den Arzneimittel-Wirkstoff enthält, wenn das NIR-Messspektrum und das NIR-Referenzspektrum in dem ersten spektralen Teilbereich innerhalb einer ersten Fehlergrenze übereinstimmen.

3. Verfahren nach Anspruch 2, **dadurch gekennzeichnet, dass** das Vergleichen des NIR-Messspektrums mit dem NIR-Referenzspektrum weiterhin Folgendes aufweist:
- Bestimmen eines zweiten spektralen Teilbereichs aus einem beim Messen des NIR-Messspektrums erfassten spektralen Gesamtbereichs, wobei der zweite spektrale Teilbereich innerhalb des spektralen Gesamtbereichs von dem ersten spektralen Bereich getrennt ist:
- Vergleichen des NIR-Messspektrums und des NIR-Referenzspektrums für den zweiten spektralen Teilbereich und
- Bestimmen, dass das zu bestimmenden Fertigarzneimittel das Arzneimittel oder den Arzneimittel-Wirkstoff enthält, wenn das NIR-Messspektrum und das NIR-Referenzspektrum in dem zweiten spektralen Teilbereich innerhalb einer zweiten Fehlergrenze übereinstimmen.

4. Verfahren nach Anspruch 3, **dadurch gekennzeichnet, dass** die zweite Fehlergrenze von der ersten Fehlergrenze verschieden ist.

5. Verfahren nach mindestens einem der vorangehenden Ansprüche, **dadurch gekennzeichnet, dass** das NIR-Messspektrum mit mehreren NIR-Referenzspektren aus einer spektralen Referenzbibliothek verglichen wird.

6. Verfahren nach mindestens einem der vorangehenden Ansprüche, **dadurch gekennzeichnet, dass** das zu bestimmende Fertigarzneimittel in einer geschlossenen Einweg-Primärverpackung.

7. Verfahren nach mindestens einem der vorangehenden Ansprüche, **dadurch gekennzeichnet, dass** das zu bestimmende Fertigarzneimittel in einem Behälter der geschlossenen Einweg-Primärverpackung, welcher aus Glas und / oder Kunststoff besteht.

8. Verfahren nach mindestens einem der vorangehenden Ansprüche, **dadurch gekennzeichnet, dass** beim Messen des NIR-Messspektrums mindestens ein optisches Messverfahren aus der folgenden Gruppe von optischen Messverfahren verwendet wird: Absorptionsmessung, Reflexionsmessung und Transmissionsmessung.

9. Verfahren nach mindestens einem der vorangehenden Ansprüche, soweit auf Anspruch 2 rückbezogen, **dadurch gekennzeichnet, dass** das zu bestimmende Fertigarzneimittel den Arzneimittel-Wirkstoff Trastuzumab-Emtansin enthaltend bestimmt wird, wenn das NIR-Messspektrum und ein weiteres NIR-Referenzspektrum in einem ersten spektralen Teilbereich von 3500 cm⁻¹ bis 5500 cm⁻¹, alternativ von 4000 cm⁻¹ bis 5000 cm⁻¹ und weiter alternativ von 4150 cm⁻¹ bis 5150 cm⁻¹ innerhalb der ersten Fehlergrenze übereinstimmen.

10. Verfahren nach mindestens einem der Ansprüche 1 bis 8, soweit auf Anspruch 2 rückbezogen, **dadurch gekennzeichnet, dass** Arzneimittel-Wirkstoff Infliximab (Originator) enthaltend bestimmt wird, wenn das NIR-Messspektrum und ein weiteres NIR-Referenzspektrum in einem ersten spektralen Teilbereich von 3500 cm⁻¹ bis 5500 cm⁻¹, alternativ von 4000 cm⁻¹ bis 5000 cm⁻¹ und weiter alternativ von 4550 cm⁻¹ bis 4750 cm⁻¹ innerhalb der ersten Fehlergrenze übereinstimmen.

11. Verfahren nach mindestens einem der Ansprüche 1 bis 8, soweit auf Anspruch 2 und 3 rückbezogen, **dadurch gekennzeichnet, dass** das zu bestimmende Fertigarzneimittel den Arzneimittel-Wirkstoff nab-Paclitaxel enthaltend bestimmt wird, wenn das NIR-Messspektrum und ein weiteres NIR-Referenzspektrum in einem ersten spektralen Teilbereich von 5600 cm⁻¹ bis 7000 cm⁻¹ und in einem zweiten spektralen Teilbereich von 4000 cm⁻¹ bis 5000 cm⁻¹ innerhalb der ersten Fehlergrenze übereinstimmen.

12. Vorrichtung zum Bestimmen eines Fertigarzneimittels, aufweisend:
- eine Messprobenaufnahme, die eingerichtet ist, als Messprobe ein zu bestimmendes Fertigarzneimittel in einer geschlossenen Primärverpackung aufzunehmen;
- einer Messeinrichtung, die eingerichtet ist, ein NIR-Messspektrum für das zu bestimmende Fertigarzneimittel durch die geschlossenen Primärverpackung hindurch aufzunehmen; und
- eine Auswerteeinrichtung, die eingerichtet ist,
- das NIR-Messspektrum mit einem NIR-Referenzspektrum zu vergleichen, welches einem Arzneimittel oder einem Arzneimittel-Wirkstoff zugeordnet ist; und
- zu bestimmen, dass das zu bestimmenden Fertigarzneimittel das Arzneimittel oder den Arzneimittel-Wirkstoff enthält, wenn das NIR-Messspektrum und das NIR-Referenzspektrum innerhalb einer Fehlergrenze übereinstimmen,
**dadurch gekennzeichnet,**
**dass** die Auswerteeinrichtung einen eine spektrale Referenzbibliothek umfassenden Speicher aufweist, wobei die Referenzbibliothek mindestens ein NIR-Referenzspektrum umfasst, welches einem als Biosimilar ausgebildeten Arzneimittel-Wirkstoff zugeordnet ist,
und **dass** die Auswerteeinrichtung eingerichtet ist, das NIR-Messspektrum mit einem NIR-Referenzspektrum zu vergleichen, welches einem als Biosimilar ausgebildeten Arzneimittel-Wirkstoff zugeordnet und in der spektralen Referenzbibliothek gespeichert ist.

## Claims

1. Method for determining a finished pharmaceutical product, said method comprising:
- providing, in a closed primary packaging, a finished pharmaceutical product to be determined;
- measuring, through the closed primary packaging, an NIR measurement spectrum for the finished pharmaceutical product to be determined;
- comparing the NIR measurement spectrum with an NIR reference spectrum which is associated with a pharmaceutical product or a pharmaceutical product active ingredient; and
- determining that the finished pharmaceutical product to be determined contains the pharmaceutical product or the pharmaceutical product active ingredient if the NIR measurement spectrum and the NIR reference spectrum match within an error limit,
**characterized in that** when comparing the NIR measurement spectrum with the NIR reference spectrum, an NIR reference spectrum is used which is associated with a pharmaceutical product active ingredient in the form of a biosimilar.

2. Method according to claim 1, **characterized in that** the comparison of the NIR measurement spectrum with the NIR reference spectrum further comprises:
- determining a first spectral sub-range from an overall spectral range detected when measuring the NIR measurement spectrum;
- comparing the NIR measurement spectrum and the NIR reference spectrum for the first spectral sub-range; and
- determining that the finished pharmaceutical product to be determined contains the pharmaceutical product or the pharmaceutical product active ingredient if the NIR measurement spectrum and the NIR reference spectrum in the first spectral sub-range match within a first error limit.

3. Method according to claim 2, **characterized in that** the comparison of the NIR measurement spectrum with the NIR reference spectrum further comprises:
- determining a second spectral sub-range from an overall spectral range detected when measuring the NIR measurement spectrum, the second spectral sub-range being separated from the first spectral range within the overall spectral range;
- comparing the NIR measurement spectrum and the NIR reference spectrum for the second spectral sub-range; and
- determining that the finished pharmaceutical product to be determined contains the pharmaceutical product or the pharmaceutical product active ingredient if the NIR measurement spectrum and the NIR reference spectrum in the second spectral sub-range match within a second error limit.

4. Method according to claim 3, **characterized in that** the second error limit is different from the first error limit.

5. Method according to at least one of the preceding claims, **characterized in that** the NIR measurement spectrum is compared with a plurality of NIR reference spectra from a spectral reference library.

6. Method according to at least one of the preceding claims, **characterized in that** the finished pharmaceutical product to be determined is in a closed disposable primary packaging.

7. Method according to at least one of the preceding claims, **characterized in that** the finished pharmaceutical product to be determined is in a container of the closed disposable primary packaging, which container consists of glass and/or plastics material.

8. Method according to at least one of the preceding claims, **characterized in that** at least one optical measurement method from the following group of optical measurement methods is used when measuring the NIR measurement spectrum:
absorption measurement, reflection measurement and transmission measurement.

9. Method according to at least one of the preceding claims, insofar as said claims refer to claim 2, **characterized in that** the finished pharmaceutical product to be determined is determined as containing the pharmaceutical product active ingredient trastuzumab emtansine if the NIR measurement spectrum and a further NIR reference spectrum in a first spectral sub-range of from 3500 cm⁻¹ to 5500 cm⁻¹, alternatively of from 4000 cm⁻¹ to 5000 cm⁻¹ and further alternatively of from 4150 cm⁻¹ to 5150 cm⁻¹, match within the first error limit.

10. Method according to at least one of claims 1 to 8, insofar as said claims refer to claim 2, **characterized in that** is determined as containing pharmaceutical product active ingredient infliximab (originator) if the NIR measurement spectrum and a further NIR reference spectrum in a first spectral sub-range of from 3500 cm⁻¹ to 5500 cm⁻¹, alternatively of from 4000 cm⁻¹ to 5000 cm⁻¹, and further alternatively of from 4550 cm⁻¹ to 4750 cm⁻¹, match within the first error limit.

11. Method according to at least one of claims 1 to 8, insofar as said claims refer to claims 2 and 3, **characterized in that** the finished pharmaceutical product to be determined is determined as containing the pharmaceutical product active ingredient nab-paclitaxel if the NIR measurement spectrum and a further NIR reference spectrum in a first spectral sub-range of from 5600 cm⁻¹ to 7000 cm⁻¹, and in a second spectral sub-range of from 4000 cm⁻¹ to 5000 cm⁻¹, match within the first error limit.

12. Device for determining a finished pharmaceutical product, comprising:
- a measurement sample receptacle that is configured to receive, as a measurement sample, a finished pharmaceutical product to be determined in a closed primary packaging;
- a measuring device that is configured to pick up, through the closed primary packaging, an NIR measurement spectrum for the finished pharmaceutical product to be determined; and
- an evaluation device that is configured
- to compare the NIR measurement spectrum with an NIR reference spectrum which is associated with a pharmaceutical product or a pharmaceutical product active ingredient; and
- to determine that the finished pharmaceutical product to be determined contains the pharmaceutical product or the pharmaceutical product active ingredient if the NIR measurement spectrum and the NIR reference spectrum match within an error limit,
**characterized in that** the evaluation device has a memory comprising a spectral reference library, the reference library comprising at least one NIR reference spectrum which is associated with a pharmaceutical product active ingredient in the form of a biosimilar, and **in that** the evaluation device is configured to compare the NIR measurement spectrum with the NIR reference spectrum which is associated with the pharmaceutical product active ingredient in the form of a biosimilar and is stored in the spectral reference library.

## Revendications

1. Procédé de détermination d'un médicament fini, comprenant :
- la disposition d'un médicament fini à déterminer dans un emballage primaire fermé ;
- la mesure d'un spectre de mesure NIR pour le médicament fini à déterminer à travers l'emballage primaire fermé ;
- la comparaison du spectre de mesure NIR à un spectre de référence NIR associé à un médicament ou à un principe actif médicamenteux ; et
- la détermination du fait que le médicament fini à déterminer contient le médicament ou le principe actif médicamenteux lorsque le spectre de mesure NIR et le spectre de référence NIR coïncident dans une limite d'erreur,
**caractérisé en ce que**, lors de la comparaison du spectre de mesure NIR au spectre de référence, un spectre de référence NIR associé à un principe actif médicamenteux réalisé sous la forme d'un médicament biosimilaire est utilisé.

2. Procédé selon la revendication 1, **caractérisé en ce que** la comparaison du spectre de mesure NIR au spectre de référence NIR comprend en outre :
- la détermination d'une première zone partielle spectrale à partir d'une zone spectrale totale détectée lors de la mesure du spectre de mesure NIR ;
- la comparaison du spectre de mesure NIR au spectre de référence NIR pour la première zone partielle spectrale, et
- la détermination du fait que le médicament fini à déterminer contient le médicament ou le principe actif médicamenteux lorsque le spectre de mesure NIR et le spectre de référence NIR coïncident dans une première limite d'erreur, dans la première zone partielle spectrale.

3. Procédé selon la revendication 2, **caractérisé en ce que** la comparaison du spectre de mesure NIR au spectre de référence NIR comprend en outre :
- la détermination d'une seconde zone partielle spectrale à partir d'une zone spectrale totale détectée lors de la mesure du spectre de mesure NIR, la seconde zone partielle spectrale étant séparée de la première zone spectrale dans la zone spectrale totale ;
- la comparaison du spectre de mesure NIR au spectre de référence NIR pour la seconde zone partielle spectrale ; et
- la détermination du fait que le médicament fini à déterminer contient le médicament ou le principe actif médicamenteux lorsque le spectre de mesure NIR et le spectre de référence NIR coïncident dans une seconde limite d'erreur, dans la seconde zone partielle spectrale.

4. Procédé selon la revendication 3, **caractérisé en ce que** la seconde limite d'erreur est différente de la première limite d'erreur.

5. Procédé selon au moins l'une des revendications précédentes, **caractérisé en ce que** le spectre de mesure NIR est comparé à une pluralité de spectres de référence NIR provenant d'une bibliothèque spectrale de références.

6. Procédé selon au moins l'une des revendications précédentes, **caractérisé en ce que** le médicament fini à déterminer est disposé dans un emballage primaire jetable fermé.

7. Procédé selon au moins l'une des revendications précédentes, **caractérisé en ce que** le médicament fini à déterminer est disposé dans un contenant de l'emballage primaire jetable fermé, lequel contenant est constitué de verre et/ou de plastique.

8. Procédé selon au moins l'une des revendications précédentes, **caractérisé en ce qu'**au moins un procédé de mesure optique du groupe de procédés de mesure optique suivant est utilisé lors de la mesure du spectre de mesure NIR :
mesure d'absorption, mesure de réflexion et mesure de transmission.

9. Procédé selon au moins l'une des revendications précédentes, dans la mesure où elle dépend de la revendication 2, **caractérisé en ce que** le médicament fini à déterminer est déterminé comme contenant le principe actif médicamenteux trastuzumab emtansine lorsque le spectre de mesure NIR et un autre spectre de référence NIR coïncident, dans la première limite d'erreur, dans une première zone partielle spectrale de 3 500 cm⁻¹ à 5 500 cm⁻¹, en variante de 4 000 cm⁻¹ à 5 000 cm⁻¹ et selon une autre variante de 4 150 cm⁻¹ à 5 150 cm⁻¹.

10. Procédé selon au moins l'une des revendications 1 à 8, dans la mesure où elle dépend de la revendication 2, **caractérisé en ce que** le médicament fini à déterminer est déterminé comme contenant le principe actif médicamenteux infliximab (médicament original) lorsque le spectre de mesure NIR et un autre spectre de référence NIR coïncident, dans la première limite d'erreur, dans une première zone partielle spectrale de 3 500 cm⁻¹ à 5 500 cm⁻¹, en variante de 4 000 cm⁻¹ à 5 000 cm⁻¹ et selon une autre variante de 4 550 cm⁻¹ à 4 750 cm⁻¹.

11. Procédé selon au moins l'une des revendications 1 à 8, dans la mesure où elle dépend des revendications 2 et 3, **caractérisé en ce que** le médicament fini à déterminer est déterminé comme contenant le principe actif médicamenteux nab-paclitaxel lorsque le spectre de mesure NIR et un autre spectre de référence NIR coïncident dans la première limite d'erreur, dans une première zone partielle spectrale de 5 600 cm⁻¹ à 7 000 cm⁻¹ et dans une seconde zone partielle spectrale de 4 000 cm⁻¹ à 5 000 cm⁻¹.

12. Dispositif de détermination d'un médicament fini, comprenant :
- un récepteur d'échantillon de mesure conçu pour recevoir, comme échantillon de mesure, un médicament fini à déterminer dans un emballage primaire fermé ;
- un dispositif de mesure conçu pour enregistrer un spectre de mesure NIR pour le médicament fini à déterminer à travers l'emballage primaire fermé ; et
- un dispositif d'évaluation conçu pour :
- comparer le spectre de mesure NIR à un spectre de référence NIR associé à un médicament ou à un principe actif médicamenteux ; et
- déterminer que le médicament fini à déterminer contient le médicament ou le principe actif médicamenteux lorsque le spectre de mesure NIR et le spectre de référence NIR coïncident dans une limite d'erreur,
**caractérisé en ce que** le dispositif d'évaluation comporte une mémoire comprenant une bibliothèque spectrale de références, la bibliothèque de références comprenant au moins un spectre de référence NIR associé à un principe actif médicamenteux réalisé sous la forme d'un médicament biosimilaire, et **en ce que** le dispositif d'évaluation est conçu pour comparer le spectre de mesure NIR à un spectre de référence NIR qui est associé à un principe actif médicamenteux réalisé sous la forme d'un médicament biosimilaire et qui est mémorisé dans la bibliothèque spectrale de références.
